Europäisches Patentamt

(19) European Patent Office

Office européen des brevets

(11) **EP 1 313 455 B1**

(12) **EUROPEAN PATENT SPECIFICATION**

(45) Date of publication and mention
of the grant of the patent:
**11.05.2005 Bulletin 2005/19**

(21) Application number: **01980290.9**

(22) Date of filing: **28.08.2001**

(51) Int Cl.⁷: $A61K\ 9/52$, $B01J\ 13/02$

(86) International application number:
**PCT/EP2001/009908**

(87) International publication number:
**WO 2002/017888 (07.03.2002 Gazette 2002/10)**

(54) **CONTROLLED AND SUSTAINED RELEASE PROPERTIES OF POLYELECTROLYTE MULTILAYER CAPSULES**

MEHRLAGIGE POLYELEKTROLYTKAPSELN MIT GESTEUERTER UND VERZÖGERTER FREISETZUNG

GESTION ET SUPPORT DE PROPRIETES DE LIBERATION DE CAPSULES MULTICOUCHES POLYELECTROLYTIQUES

(84) Designated Contracting States:
**AT BE CH CY DE DK ES FI FR GB GR IE IT LI LU MC NL PT SE TR**

(30) Priority: **28.08.2000 EP 00118615**
**23.05.2001 EP 01112600**

(43) Date of publication of application:
**28.05.2003 Bulletin 2003/22**

(73) Proprietor: **Max-Planck-Gesellschaft zur Förderung der Wissenschaften e.V.**
**80539 München (DE)**

(72) Inventors:
• **ANTIPOV, Alexei**
**14476 Golm (DE)**
• **VIEIRA, Euridice**
**14424 Potsdam (DE)**
• **IBARZ, Gemma**
**14424 Potsdam (DE)**
• **SUKHORUKOV, Gleb**
**14471 Potsdam (DE)**
• **DÄHNE, Lars**
**12587 Berlin (DE)**
• **GAO, Changyou**
**14476 Golm (DE)**
• **DONATH, Edwin**
**16845 Giesenhorst (DE)**
• **MÖHWALD, Helmuth**
**55411 Bingen (DE)**

(74) Representative: **Dey, Michael, Dipl.-Chem. Dr. et al**
**Postfach 860 820**
**81635 München (DE)**

(56) References cited:
**WO-A-01/37803**          **WO-A-02/09865**
**WO-A-99/47252**          **DE-A- 19 519 804**

**Description**

The present invention relates to methods of controlling the permeability of micro containers for drug encapsulation and release, and especially the control of sustained release properties of polyelectrolyte multilayer capsules.

A major task in the development of advanced drug formulations deals with the elaboration of delivering systems providing sustained release of bioactive materials. Mostly, these delivering systems comprise polymer particles in the size range of $10^2$ to $10^5$ nm. The drug molecules are embedded in polymer matrices or in core-shell structures. In the latter the shell degradation rate determines the release rate of the bioactive core material.

In principle, a shell around the active core can be fabricated by adsorption of polymers or biopolymers onto the drug particle surface [1] or by adsorption of the monomers with subsequent polymerization at the interface [2-4]. The composition of the shell may additionally provide certain functionalities. It may be adjusted to facilitate the interaction of the core with the solvent or to add certain desired chemical properties. The shell may also have magnetic, optical, conductive, or targeting properties for directing and manipulating the core containing bioactive material.

Recently, a novel type of shell structures constituting polymer capsules has been introduced [5-7]. These novel hollow polymeric capsules have a predetermined size in the sub-micron and micron range and tunable wall properties [7,13-15]. These capsules are fabricated by means of layer-by-layer (LbL) assembling of polyelectrolytes onto colloidal particles with subsequent removal of the colloidal core. The layer-by-layer (LbL) assembling is performed by alternating adsorption of oppositely charged species, such as polyelectrolytes [16,1] onto the surface of colloidal particles. The driving force for LbL adsorption is the electrostatic attraction between the incoming polymer and the surface.

Various cores, e.g. organic or inorganic materials, biological cells, drug crystals or emulsion droplets, ranging in size from about 60 nm to tens of microns have been utilized as templates for multilayer formation by the LbL technique. Up to now a variety of different substances, such as synthetic and natural polyelectrolytes, biopolymers, proteins, nucleic acids, magnetic and fluorescent inorganic nanoparticles, lipids, etc. were employed as layer constituents to build the multilayer shell on colloidal particles. The thickness of the shell walls depends on the conditions of its preparation. It can be tuned in the nanometer range. The thickness of the multilayer films on colloidal particles can be adjusted in the nanometer range e.g. by adsorption of varying numbers of layers. It was established [8, 9] that the capsule walls have semipermeable properties. They are permeable for small molecules such as dyes and ions while they exclude compounds with a higher molecular weight [17,9].

There is a variety of materials, the encapsulation of which is desirable for application in different areas of technology, such as catalysis, cosmetics, medicine, biotechnology, nutrition and others.

One possible approach to load capsules with polymers is to embed the desired polymers within the inner layers of the shell structure while forming the capsules and to desorb polyelectrolytes, e.g. multivalent ions, from inner layers of empty shells into their interior [18]. Extraction of these ions results then in the release of the polymers into the capsule interior.

Another approach is to directly synthesize polymers inside the capsules by taking advantage of the permselectivity of the capsule walls [19]. While capsules can be successfully loaded thereby these methods have only limited applicability with regard to the polymer species and often harsh conditions have to used for core dissolution or polymerization, being e.g. a low pH, oxidizing agents or organic solvents, which are used for core decomposition, or an elevated temperature during synthesis.

It would be desirable to provide systems having controllable or adjustable loading as well as release properties. In particular a method would be favourable which allows the loading or release of materials into and from capsules by modifying the capsule wall permeability. Further, for most applications a defined and controllable permeability of the capsule wall is required in order to control the process of loading the capsules as well as any subsequent release under specific environmental conditions. Due to the fact that the loading is preferably fast, but the release should be in most applications slow, it is further desired that the permeability is switchable.

It was therefore an object of the present invention to provide methods to influence, vary or switch properties of capsule walls. Another object of the invention was to provide means to introduce macromolecules into the capsules and to switch and control capsule permeability for them.

These objects are solved by a process for controlling the permeability of polyelectrolyte multilayer capsules by variation of the conditions during the use of the capsules, characterized in that at least one of the reaction conditions of pH, temperature, salt concentration, ion composition, ion concentration, ionic strength, solvent composition or solvent concentration is varied.

According to the invention the permeability of polyelectrolyte multilayer capsules can be determined, varied or/and controlled by parameters of the environment of the capsules, the use of the capsules, media, in which the capsules are contained or into which the capsules are transported. It was found that the permeability to high molecular weight compounds as well as low molecular weight compounds can be adjusted according to the needs in different applications.

As used herein high molecular weight compounds or macromolecules are molecules having a molecular weight of at least 30 000 Da, more preferably at least 50 000 Da and most preferably at least 70 000 Da. Low molecular weight compounds or small molecules are molecules having a molecular weight of less than 10 000 Da, preferably less than 5 000 Da and more preferably less than 1 000 Da.

The permeability control according to the invention offers a unique tool for entrapping molecules within capsules and releasing them in a predetermined manner, e.g. over an extended period of time or at a desired, predetermined site or time point.

The process according to the invention enables particularly a reversible amendment of the permeability of capsules. This enables specifically charging the capsules with desired active substances or specifically releasing active substances entrapped in the capsules, respectively, by amending the environmental conditions of the capsules in a simple way. Thus, e.g. a permeability increase makes it possible to later charge the finished capsules with active substances. Such an "open condition", wherein active substances can permeate through the capsule walls is present according to the invention when over 50%, more preferably over 70% and most preferably over 90% of the capsules are permeable. After the capsules have been charged, e.g. during storage or transport, the permeability of the capsule wall to the entrapped active substance can be reduced by adjusting of suitable conditions so that no active substance can leave the capsules. Such a "closed condition" of the capsules, however, at the same time prevents that further, possibly undesired substances can enter the capsules. At the desired time and site of release, respectively, the active agent can be released in a defined way, e.g. delayed, by increasing the permeability of the capsule walls. Further, it is possible to obtain a release in certain compartments of cells or certain areas of an organism by using capsule walls exhibiting high permeability to the entrapped active substance under conditions prevailent in the desired tissue.

According to the invention the permeability of capsule wall and thereby the incorporation or exclusion of macromolecules can be tuned by environmental conditions in a defined manner. Capsules composed of polyelectrolytes, the charge of which depends on the pH, can be used for a pH-controlled uptake and release of macromolecules.

The practical use of polyelectrolyte capsules for sustained drug release requires quantitative data on the permeation of small molecules through polyelectrolyte walls, which are presented herein.

In the light of sustained release it would be advantageous to be able to decrease the layer permeability for small polar molecules once they are encapsulated. One possible way to approach this goal is the use of lipids as a layer constituent [9, 10]. Herein, the formation of thicker capsule walls is presented in detail being a favourable and easy way to decrease permeation. It was found that increasing the layer number will decrease penetration of the shells by encapsulated material or molecules.

To verify this approach fluorescein microparticles were covered with a different number of polyelectrolyte layers. Dyes like fluorescein can be considered as model substances for a large class of drugs. Afterwards core dissolution was initiated by a pH change and monitored by the increasing fluorescence in the bulk.

It was found that polyelectrolyte multilayer shells assembled around cores consisting of low molecular weight compounds provide barrier properties for release under conditions where the core is dissolved. This finding is a novel approach for fabrication of systems with prolongated and controlled release properties. The release can be adjusted with the number of assembled polyelectrolyte layers. The capsule permeability for low molecular weight compounds was found to depend strongly on the number of polyelectrolyte layers in the capsule.

A large variety of synthetic polyelectrolytes with different properties, lipids, and polysaccharides have been already used for multilayer assembly [9, 12]. This provides many possibilities to tune the release properties of the shells together with ensuring biocompatibility and possibility of using various cores. The assembling of shells by LbL technique opens new pathways for biotechnological applications, where controlled and sustained release of a substance is required. Many problems connected with drug formulation, release, and delivery, controlling the concentration in the organism and periodicity of its reception can be solved by the formation of shells on precipitates and nanocrystals. Further it is not difficult to add to the polyelectrolyte layer targeting properties. This way the affinity of polyelectrolyte multilayer coated drugs to specific or injured tissues can be increased.

In the following the release properties of capsules with regard to the number of layers forming the capsule wall are discussed in more quantitative terms using fluorescein as model substance (MW $\sim$ 350 Da).

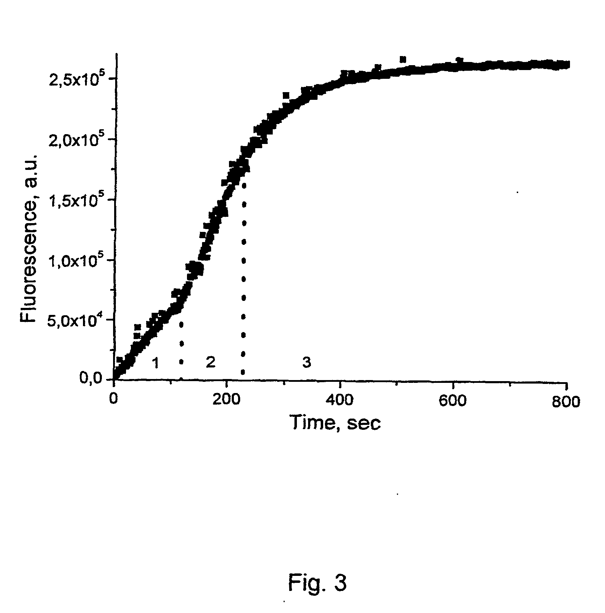

The permeation of a molecule such as fluorescein through the shell wall is described by its permeability (P). Equation (1) combines a flux (J) with parameters of the system and the rate of change of fluorescein concentration, $c^e$, in the bulk. When this rate is constant one may easily calculate the permeability (P) from the slope of the fluorescence increase (part 2 of Fig.3).

$$\frac{dc^e}{dt} V_0 = J = P \cdot (c^i - c^e) \cdot S \qquad (1)$$

where $V_0$ is the volume of solution, J is the fluorescein flux through the capsule walls with the total surface area of S,

and ($c^i$ - $c^e$) is the difference of the fluorescein concentration inside ($c^i$) and outside ($c^e$) the capsules.

As long as a core of solid fluorescein is present within the capsules, the interior of the capsules contains a saturated fluorescein solution, $c^s$, of 25 mg/ml. Hence, the concentration difference at the beginning of the process in the right side of Eq.1 can be safely replaced by $c^i = c^s$. The capsules were assumed spherical with an average diameter 5 $\mu$m. The permeability can thus be calculated from

$$P = \frac{dc/dt \cdot V_0}{c^s \cdot S} \tag{2}$$

For 8 to 18 layers the permeability value was in the order of $10^{-8}$ m/s. Assuming single polyelectrolyte layer thickness of 2 nm the permeability can be converted into a diffusion coefficient (D) by means of multiplying the permeability with the shell wall thickness. The calculated diffusion coefficients are in the order of $10^{-15}$ m$^2$/s.

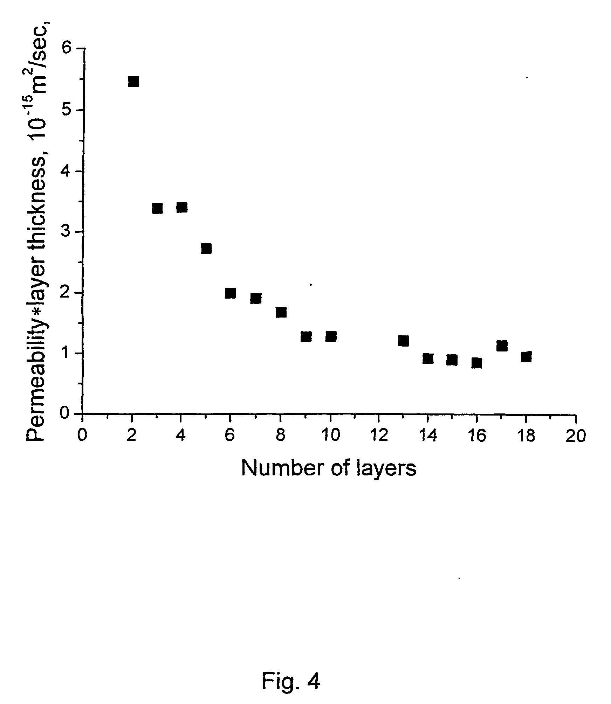

If the permeability of the polyelectrolyte multilayer is provided by diffusion through the entangled polymer network, it should scale with the inverse of the layer thickness. The behavior of the permeability times thickness as a function of the number of layers is shown in Figure 4. As can be seen, the permeability decreases with increasing layer number much faster than expected from a straightforward thickness increase. Only from approximately 8 layers onwards the permeability multiplied by the shell thickness becomes a constant indicating that the permeability is now controlled by the thickness increase, for example, the diffusion limiting region is the polyelectrolyte layer. This finding is consistent with the earlier observations [11] where it was shown that the conformation of the first eight layers differs from that of further assembled layers. These deeper layers are more densed resulting in a fivefold reduction of the estimated diffusion coefficient as can be inferred in Fig. 4.

A permeability coefficient might also be calculated from the release profile at the third stage of the fluorescein release curve. It can be expected that the time dependence of the release at this stage is exponential:

$$c^e(t) \sim \left( 1 - e^{-P \frac{\bar{S}}{V} t} \right), \tag{3}$$

where $\bar{S}$ and $V$ represent the surface and the volume of an average capsule. However, the permeability estimated from this equation was one order less than the one calculated from the slope of the linear region. There are currently two possible explanations for this discrepancy. Either the polydispersity of the particles contributes to an apparent prolonged release, because the characteristic time release $V/\bar{S}P$ increases linearly with the particle radius. Or, the relaxation of the osmotic stress towards the end of release may reduce the permeability.

In the following the permeation mechanism is discussed in more detail. One may distinguish between diffusion through water filled pores and a "bulk" diffusion mechanism through homogeneous phase of polyelectrolyte multilayer shell. For thinner walls the drastic dependence on shell thickness may be explained either by existence of pores that are successively closed by further layer deposition or by thickness dependent diffusion coefficient. The latter was indeed observed with permeation studied by planar polyelectrolyte films where typical values of D between $10^{-18}$ and $10^{-20}$ m$^2$/sec were derived [11]. In order to compare our results with those of the previous study we should remark the following differences.

-     According to the method described in [11] the films were prepared by drying after each adsorption step which is not possible during particle coating and which leads to a denser film.

-     The measurement consisted in depositing a dye probe in a defined depth into the film and observing time dependent fluorescence changes due to quencher diffusion. For sufficiently small pore concentration, which is surely the case here, this technique is only sensitive to "bulk" diffusion and will not reflect the permeation through pores.

Thus one may expect that the diffusion coefficients derived from permeability data are larger than values measured in [11]. Still, this cannot explain the difference of 3-4 orders of magnitude and therefore they more probably correspond to diffusion through pores. Comparing the diffusion coefficient D with that in bulk water ($\sim 10^{-10}$ m$^2$/s) which is an upper limit for the diffusion in the pore volume we may estimate the fraction of pore volume inside the walls larger than $10^{-5}$.

Next a possible mechanism of pore formation is adressed. A destabilization of the wall may be expected, since on increasing the pH towards 8 the aminogroups of PAH may deprotonate and this may result in pore formation within the multilayers. Another possibility of pore formation is the osmotic pressure due to the water coming to the interior as a result of fluorescein core dissolution. The related hydrostatic pressure difference creates a tension in the wall which may widen existing or create new pores. As seen on Fig.3 (part 1), especially for bigger numbers of layers, in the beginning of dissolution the release is sustained. It is assumed that the polyelectrolyte shell suffering osmotic pressure from inside resists release of fluorescein until the fluorescein molecules develop a path out of capsules. The pores are formed as a result of this rearrangement of polyelectrolyte multilayers. It should be noted that templates and the resulting capsules have different diameters and also heterogeneous wall thicknesses. The diffusion coefficient is thus an average but there may still be larger templates with slower release profile.

In summary, it can be noted that by increasing the number of layers of polyelectrolyte capsule walls the permeability of the capsules can be reduced. For a delayed release preferably capsules having $\geq 8$, more preferably $\geq 9$ and most preferably $\geq 10$ layers are used.

The invention relates to a novel approach for encapsulating materials and molecules, such as macromolecules, biopolymers, drugs etc., in pre-formed hollow polyelectrolyte capsules. According to this embodiment of the invention the material or/and molecules can be introduced in hollow capsules rather than forming capsules around the materials/molecules.

The properties and structure of polyelectrolyte multilayers are found to be sensitive to a variety of physical and chemical conditions of the surrounding media. In particular, the pH is one of the physico-chemical parameters, which influences the state of the inter-polyelectrolyte complex [20,21], especially in the case, if the charge of one polyelectrolyte in the complex depends on the pH. The polyelectrolyte pair, poly(styrene sulfonate) (PSS) and the relatively weak polycation poly(allylamine hydrochloride) (PAH), has been most extensively used for producing multilayer films on flat and colloidal surfaces (1) and serves as exemplary polyelectrolyte pair for illustrating the invention. However, the invention is not limited to this specific polyelectrolyte pair.

By using different polyelectrolytes, the permeability can be further modified. The combination of cationic and anionic polymers results in almost unlimited variation possibilities. Characteristic values, such as polarity and polymeric rigidity can be adjusted via the chemical composition and have a specific effect on different substance classes. For example a reduction of the polarity results in a higher hydrophobicity, which decreases the permeability for polar, water soluble substances, whereas the permeability for unpolar, oil soluble substances is enhanced.

In addition, the permeability behaviour of the capsules can be strongly modified via the molecular weight and the degree of branching of the polyelectrolytes used. The molecular weight is advantageously set between 10 000 and 500 000 g/mol, wherein generally higher molecular weights and increasing branching lead to higher capsule stability and lower permeability.

By varying the pH of the medium, the permeability of the walls of microcapsules can be varied. While it is not intended to be bound to a specific mechanism of the pH-induced permeability change, it is supposed that changes of the polyelectrolyte charge upon pH variation are able to induce pore formation (22) or loosen the polyelectrolyte network, thus enabling polymer penetration. An influence of the ion concentration going along with the pH change may also contribute to the permeability change.

This possibility of switching the capsule walls between an open and closed state provides a convenient and efficient tool to control the uptake and release of molecules, in particular polymers, biopolymers and nanoparticles. For instance, the capsules might be loaded at low pH and after increasing the pH the material is captured inside. For release the pH can be slightly decreased again, whereby the kinetics of release can be tuned by the pH. The herein demonstrated possibility of controlling loading and release of macromolecules into and from polyelectrolyte capsules allows for widespread application. The described pH-induced permeability change of the polyelectrolyte network in the film is a general mechanism for modifying the permeability of polyelectrolyte multilayers and is not limited to a specific composition of the shell. Investigations on flat polyelectrolyte films made from weak polyelectrolytes have shown that pores can be created by changing the pH or the salt concentration.

We have found that permeability of capsules composed of polyelectrolytes, in particular of weak polyelectrolytes, strongly depends on the pH. Being impermeable for macromolecules at a pH when the charges of two polyelectrolytes forming the capsule wall are compensated, capsules are open at a pH, at which a weak polyelectrolyte is completely charged. This dependence gives an opportunity to encapsulate polymers by opening and closing a. capsule by means of pH. By means of AFM at the open state pores ranging in size from 100 to 300 nm were observed in the capsule wall. A further remarkable feature of the process of pore formation by pH variations is that the pore formation or increased permeability is reversible.

Further permeability of the capsules inversely depends on the number of layers they consist of. Employment of both these factors gives a possibility to regulate capsule release properties for low as well as for high molecular weight compounds. Fluorescein particles, stable at acidic pH can be used as core. They can be covered with different numbers of polyelectrolyte layers. The kinetics of fluorescein dissolving and release was measured at base pH. It was found that time of core decomposition depends strongly on the capsule wall thickness. During the dissolving a sustained release of fluorescein was observed.

Permeability control by means of pH and capsule wall thickness is proposed as a new approach for controlled uptake and release of different substances into and from capsules. It opens avenues for using polyelectrolyte capsules for many applications, such as drug cariers, microreactors and so on.

In summary, it can be stated that by increasing the pH a reduced capsule permeability can be achieved and by reducing the pH an increased capsule permeability can be achieved, whereby this process is reversible. It was found that capsule walls particularly at a pH of > 8, more preferably at a pH of > 10 are impermeable (closed condition), whereas at a pH of < 6, particularly of < 4 a permeability (open condition) of the capsule walls can be achieved. The change in pH represents a simple way for adjusting the permeability and by amending the surrounding medium makes it possible to provide for a permeability switch for the capsules.

This application also presents a novel, simple and very gentle method for targeted encapsulation and release of sensitive, macromolecules by controlling the capsule wall permeability by the salt concentration. Salts have an immense importance in the living organisms. The salt concentration in the cytoplasm and in the extra cellular medium is strongly regulated by the permeability of the cell membranes, by osmosis and by active transport via energy driven pumps. The physiological concentration of sodium chloride in plasma is about 0.15 M, other ions, such as potassium or calcium are crucial for signal transduction across the membrane and inside the cell.

The permeability of hollow polyelectrolyte capsules, preferably of capsules made from PAH/PSS can be reversibly switched between an open and closed state by varying the salt concentration in a narrow concentration range between 0.5 ... $2 \times 10^{-2}$ M/l. The increase of the salt concentration leads to a reversible opening of the capsule wall for large molecules of MW above 50 000 Da, preferably above 70 000 Da.

The absence of pores in scanning force microscopy images, the relatively small permeability and the step-like descrease of the Förster resonance energy transfer in the wall with increasing salt concentration indicates a weakening of the electrostatic interactions between the polyelectrolytes and a subsequent swelling connected with a much better diffusion of the polymers. Salt triggered permeability is a very easy, important, and effective way for the encapsulation and release of, especially, sensitive macromolecules such as enzymes, proteins, or DNA in micro- and nanocontainers.

In summary, it can be noted that by increasing the salt concentration an increase of the permeability of the capsule walls can be achieved, whereas reducing the salt concentration causes a reduction of the permeability (closed condition). Preferably the capsule walls are made permeable by a salt concentration of at least $2 \times 10^{-2}$ M, more preferably of at least $3 \times 10^{-2}$ M and most preferably of at least $1 \times 10^{-1}$ M. If a closed condition of the capsules is desired, the surrounding conditions are adjusted to a salt concentration of at most $5 \times 10^{-3}$ M, more preferably of at most $1 \times 10^{-3}$ M and most preferably of at most $1 \times 10^{-4}$ M.

Suitable salts according to the invention are all heteropolar compounds comprising at least one anion and at least one cation. The anions and cations can be singly charged or multiply charged ions. For adjusting the permeability preferably inorganic salts are used, particularly metal salts, such as metal halogenides, particularly alkali metal halogenides. Examples of particularly preferred salts are salts containing an alkali metal cation, particularly $Li^+$, $Na^+$, $K^+$, an earth alkali metal cation, particularly $Mg^{2+}$, $Ca^{2+}$ or a different metal cation, e.g. $Al^{3+}$, an iron ion, etc. and an anion, e.g. a halogenide anion, or a different anorganic anion. The metals may also be replaced by other positively charged groups, e.g. an ammonium group; a sulfonium group or a phosphonium group. However, it is also possible to-use organic salts, particularly salts containing organic anions.

Apart from the salt concentration, the permeability can also be adjusted by the ion concentration and ion composition of the environment, respectively, wherein also here it was detected that for higher ion concentrations a permeability is achieved, whereas for low ion concentrations an impermeable capsule wall was obtained.

The ion composition particularly plays a role, since the permeability of the capsules is influenced by interactions between ions of the surrounding medium and the charges of the polyelectrolyte shells. By suitably selecting the ions of the surrounding medium, e.g. ions forming complexes with the polyelectrolytes of the capusule shells, the permeability can be adjusted.

The dependence of permeability on salt concentration, ion composition and ion concentration can be particularly used advantageously by administering encapsulated active substances to organisms, e.g. mammals and particularly humans. Therefor capsules can first be charged with active substances, wherein in vitro the capsule walls are made permeable to the active substance with the help of the above described measures. Then the encapsulated active substances can be stored in a stabil form, e.g. by adjusting a salt concentration of the storage medium to $< 5 \times 10^{-3}$ M. This can be done e.g. by storage in distilled, perferably in sterile distilled water. The administration of charged capsules is accompanied by an amendment of the surrounding medium, wherein, as already stated above, the physiological concentration of sodium chloride in plasma is about 0.15 M. Under these surrounding conditions the capsules are permeable to the active substance so that the active substance is released in the target tissue.

The invention also relates to a simple method for the polyelectrolyte capsules allowing opening and closing of the wall for large polymers but also for small organic molecules and to control the permeability in a simple way by temperature treatment.

It has been shown, that the permeability of thin polyelectrolyte walls can be switched easily by heat. This is probably caused by an temporarily swelling of the wall due to stronger dissociation of the cation-anion bonds at higher temperature, followed by hydration of the formed charges and entanglement of the polymer chains. The reversibility of this process provides an excellent tool for loading hollow polyelectrolyte capsules with large macromolecules, polymers or nanoparticles for drug encapsulation as well as for the controlled and intelligent release of active substances. For example, in the agriculture many pests are active mainly at high temperatures. Encapsulation of pesticides in such capsules can ensure a release only at hot and sunny days. Or, in the new generation of intelligent clothes, deodorants will be released only in case of strong swetting.

Preferably at a temperature of up to 50°C, more preferably up to 40°C, the permeability is low (closed condition), whereas at a temperature of above 50°C, particularly of at least 60°C, permeable capsules (open condition) are obtained. By increasing the temperature in a simple way a permeability increase can be achieved.

In a further embodiment of the invention the permeability of polyelectrolyte multilayer capsules is amended by varying the solvent composition or solvent concentration. Thus, the release behaviour can be adjusted via the solution, wherein as solution e.g. polar solutions, particularly water or/and polar solutions or mixtures thereof can be used. Particularly suitable solutions for adjusting the release behaviour are alcohol/water mixtures, wherein the alcohol content, e.g. methanol, ethanol or propanol, can be adapted to the individual application.

Further, the permeability of polyelectrolyte multilayer capsules can further be influenced by adding surfactants, such as sodium dodecylsulfate (SDS) or dipalmitoyl-DL-$\alpha$-phosphatidyl choline (DPPC) to the surrounding medium or as coating for the core used in capsule production.

The permeability of polyelectrolyte multilayer capsules can also be influenced by light. Photosensitive groups in the electrolyte layer, such as azo compounds or organic bisazides can be modified chemically by light, which may lead e.g. to an increase of the permeability or to a reduction of the permeability, if the photosensitve compositions are selected which cross-link by exposure to light.

As explained above, according to the invention it is possible to insert further components into the polyelectrolyte layers in order to obtain a broad modification of the permeability. The further components can e.g. be bound covalently to the polyelectrolyte or be independent molecules or particles, which are only embedded in the polyelectrolyte layers. The further components can be incorporated together with the polyelectrolyte or as independent units by using various methods, preferably the layer-by-layer method, but also a single step method or subsequent diffusion.

The microcapsules, in particular hollow microcapsules, whose permeability can be controlled according to the invention preferably can be fabricated by alternating deposition of oppositely charged polyelectrolytes on soluble colloidal templates. The polyelectrolyte capsules can e.g. be prepared by covering templates, ranging e.g. from 60 nm, preferably from 100 nm, to 10 µm, preferably to 2 µm in size, with alternating layers of polycations and polyanions [6,7]. After formation of a polyelectrolyte wall of sufficient thickness, the templates can be dissolved and hollow capsules are obtained. The templates can e.g. be removed by changing the pH or by oxidative decomposition of the template. This is possible due to the remarkable property of the capsule wall to be permeable for small molecules having a molecular weight of less than 10 000, preferably less than 5 000 and particularly less than 1 000 but not for polymers. The template determines size and shape of the capsule. Monodisperse capsules in the range from 500 nm to 10 µm can be prepared. Suitable capsules and their production and composition are described, e.g. in WO99/47252, WO99/47253, WO00/03797 and WO00/77281.

A remarkable feature of the capsule wall is that their properties can be tuned within wide ranges by varying the layer material and/or number or/and by varying environmental conditions such as pH, salt concentration, ion strength, ion composition, solvent concentration, solvent composition or/and temperature. These capsules offer broad perspectives in nanoscale encapsulation of drugs, enzymes, DNA, minerals, dyes, polymers, proteins and/or other active macromolecules.

The capsules are preferably fabricated by alternate adsorption of oppositely charged polyelectrolytes onto the surface of colloioal particles. Different cores having a size varying from 0.06 preferably from 0.1 to 10 µm, such as inorganic colloidal particles, biological cells, protein aggregates, drug nanocrystals can be used. Hollow capsules can be produced by subsequently dissolving the core.

The permeability of capsules is of essential interest because of its significant importance in diverse areas, relating e.g. to biotechnology, medicine, food industry, etc.

The invention shall be further illustrated by the following examples and figures:

Fig. 1: Scheme of the polyelectrolyte multilayer deposition process and of the subsequent core dissolution. The initial steps (A-D) involve stepwise shell formation on a fluorescein core. After the desired number of polyelectrolyte layers is deposited the coated particles are exposed to pH 8 (E) and core dissolution with fluorescein penetration into the bulk is initiated resulting finally in fully dissolved cores and remaining empty capsules (F).

Fig. 2: Fluorescence increase upon time, obtained by dissolving fluorescein particles covered with shells of different thickness (9, 13, 15, and 18 layers).

Fig. 3: Three stages of fluorescein core dissolution covered with 17 PSS/PAH layers.

Fig. 4: Fluorescein diffusion as a function of layer number.

Fig. 5: Permeation and encapsulation of FITC-dextran (M.w. 75 000) into polyelectrolyte multilayer capsules. Left - pH = 10, center - pH = 3, right - pH increased to 10 after the capsules were loaded with FITC-dextran at pH = 3. The bulk FITC-dextran was removed by washings at pH = 10. Top - scheme, center - confocal images of the capsules, bottom - fluorescence profile along the line indicated in the confocal images.

Fig. 6: Confocal Laser Scanning Microscopy images showing: a) capsules for which the washings and the core dissolution was performed in the presence of 0.05 M NaCl; b) capsules which were washed with pure water after each deposition step; 24 h incubation in a $10^{-3}$ M PAH-Rho solution; c) same procedure as in b) but incubation in presence of $10^{-2}$ M salt.

Fig. 7: Efficiency of the Förster Resonance Energy Transfer in capsules containing the 10th layer PAH-fluo and the 12th layer PAH-rho in dependence on the salt concentration. The inset shows a typical fluorescence spectrum of these capsules. Excitation was set at 495 nm.

Fig. 8: Time-dependent decrease of the FRET efficiency as a function of the salt concentration.

Fig. 9a): Capsules, loaded with 5 x $10^{-3}$ M PAH-rho according to the protocol shown in Fig. 9b) Same capsules after release of the encapsulated PAH-rho induced by a 3 x $10^{-2}$ M salt solution.

Fig. 10: Scheme of encapsulation and release of macromolecules by switching the permeability salt concentrations changes.

Fig. 11 a): CLSM image of capsules in presence of $10^{-3}$ M Fluorescein-PAH (MW 70 000 g/mol, polymer exclusion); b) CLSM image of capsules in presence of $10^{-3}$ M Rhodamine-Dextran (MW 50 000 g/mol, accumulation);

Fig. 12a): CLSM image of capsules in presence of $10^{-3}$ M Rhodamin-PAH (MW 70 000 g/mol) at 23°C; b) encapsulated Rhodamine-PAH in capsules after heating the solution for 20 min to 60°C and subsequent washing with water.

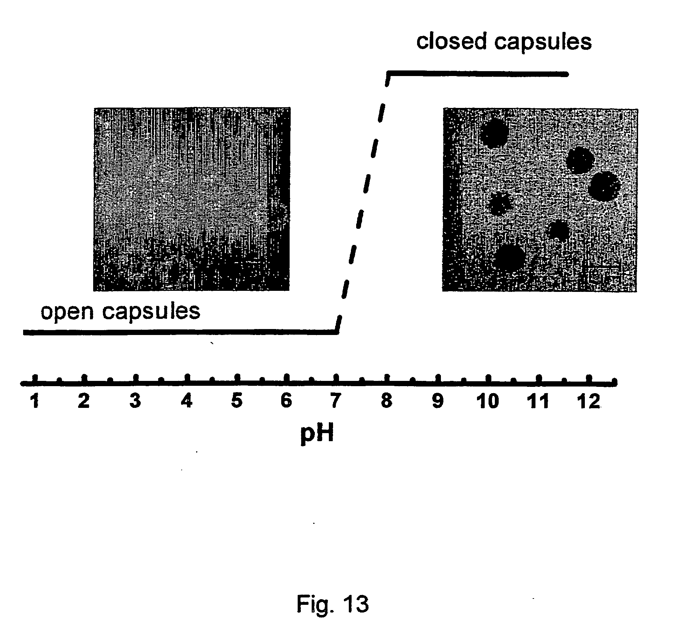

Fig. 13: Permeability of capsules composed of 8 layers of PSS-PAH for a labeled dextran, molecular weight 77 000 at a low and high pH.

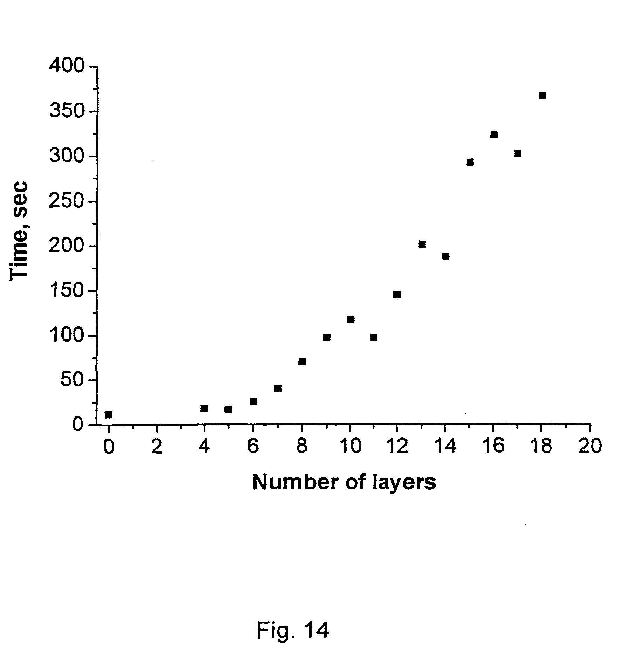

Fig. 14: Characteristic time of dissolving of fluorescein core, covered with different number of PSS-PAH polyelectrolyte layers.

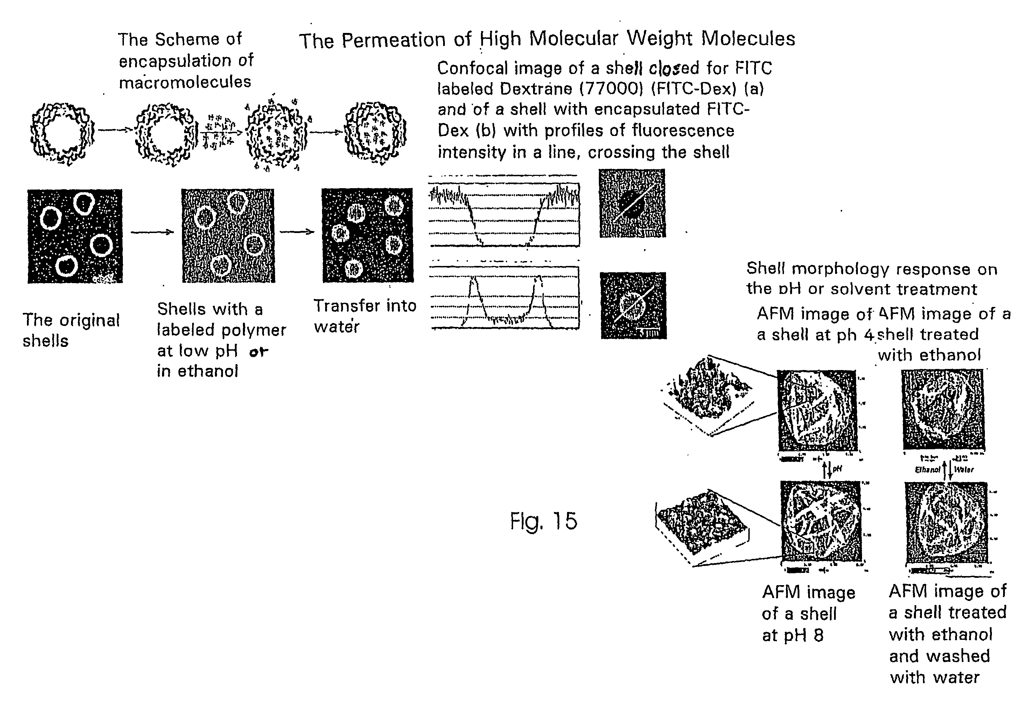

Fig. 15: The permeation of high molecular weight molecules.

Table 1: Percentage of filled capsules in solutions of PAH-rho, 5 x $10^{-3}$ M, after 24 hours incubation time and in presence of different salt concentrations.

Examples

Example 1: Varying number of polyelectrolyte layers

Materials

Polyelectrolytes. Sodium poly(styrene sulfonate) (Na-PSS, MW ~70 000), poly(allylamine hydrochloride) (PAH, MW ~50 000), and fluorescein, sodium salt, were obtained from Aldrich. Ethanol, sodium chloride, boric and hydrochloric acid were purchased from Sigma. All materials were used without further purification.
The water used in all experiments was prepared in a three stage Millipore Milli-Q Plus 185 purification system and had a resistivity higher then 18.2 MΩ cm.

Methods

Fluorescein particles were prepared by addition of one part of ethanol and four parts of hydrochloric acid, pH 2, to one part of 15 mg/ml aqueous fluorescein solution. The particle size was measured by optical microscopy. The 4-9 μm in diameter fluorescein particles were washed in hydrochloric acid by 5 repeated centrifugation circles at 450 g. To prevent preliminary core decomposition all further multilayer deposition and washings were performed in hydrochloric acid at pH 2.

Polyelectrolyte multilayer assembly. The multilayer assembly was accomplished by adsorption of polyelectrolytes at a monomer concentration of $10^{-2}$ M in 0.5 M NaCl, pH 2. Oppositely charged polyelectrolyte species were subsequently added to the suspension of fluorescein particles followed by repeated centrifugation cycles in hydrochloric acid [7]. Fluorescein particles were allowed to interact with polyelectrolyte solution for 15 minutes. Fluorescein particles were centrifuged at 700 g for 10 minutes. Gentle shaking followed by 1 minute ultrasonication was used to disperse particles after centrifugation.

Fluorescence spectroscopy. The core dissolving was conducted in $H_3BO_3$-NaCl-NaOH buffer, pH 8. The kinetics was followed by recording the time dependence of the emission at 522 nm. Excitation was set at 488 nm.

Confocal microscopy. Confocal images of capsules after dissolving the core were obtained by means of a Leica confocal scanning system. A 100 x oil immersion objective with a numerical aperture of 1.4 was used.

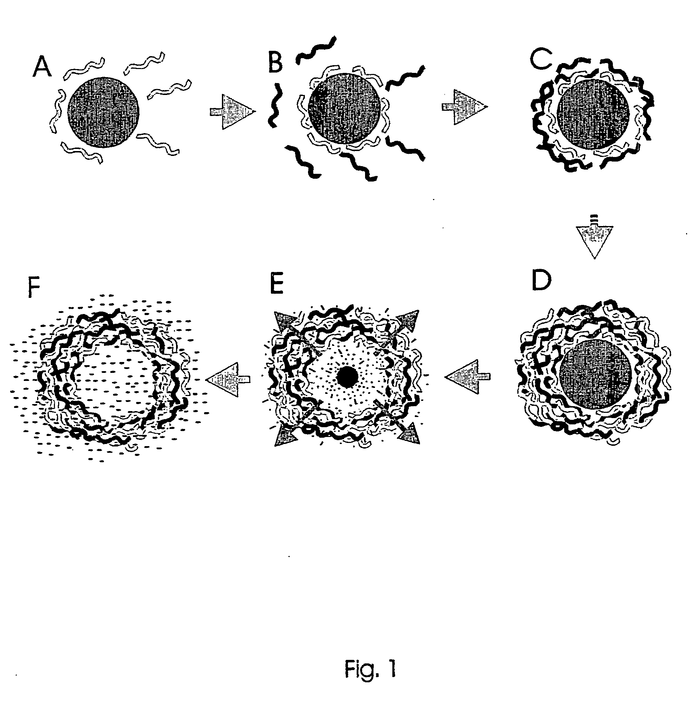

Figure 1 provides the scheme of fluorescein particles encapsulation and release. After LbL adsorption (Fig. 1A-1D) core dissolution is initiated by changing the pH from pH 2 to pH 8 (Fig. 1 E) and completed after a certain period of time (Fig. 1F).

Fluorescein particles rapidly dissolve at pH 8. The idea was thus to slow down the rate of core dissolving by covering the particles with a polyelectrolyte multilayer. Shells walls consisting of a different number of layers were fabricated and examined with regard to their fluorescein permeability behavior. Fluorescence spectroscopy is a convenient tool for the determination of the core dissolving rate because the fluorescence of the core is completely suppressed as a consequence of the self-quenching of the dye. Upon releasing the dye into the bulk the fluorescence intensity increases. Thus the rate of cores dissolving can be directly followed by measuring the fluorescence increase in the sample.

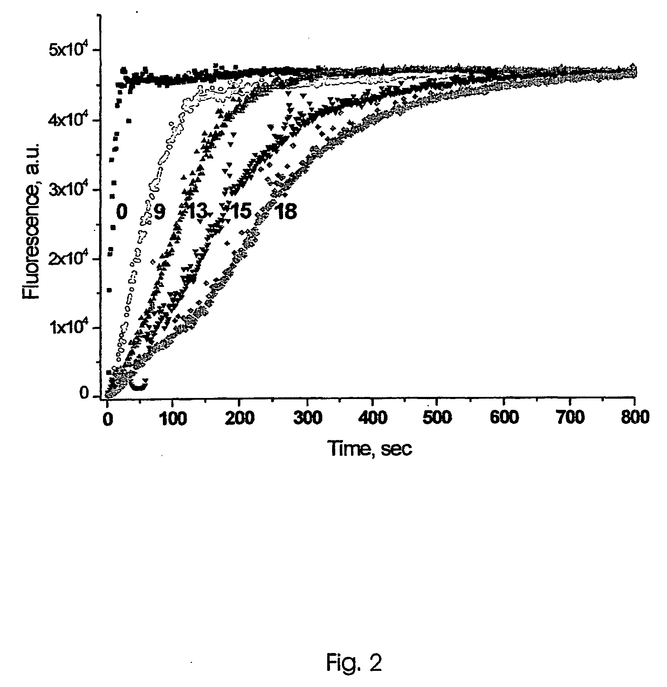

In Fig. 2 typical time-dependent fluorescence curves obtained by switching the pH to 8 are shown. Fluorescein particles covered by layers of different thickness (9, 13, 15, and 18 layers) are compared with the control demonstrating the dissolving of naked fluorescein particles.

As shown in Figure 3, after a comparatively short induction period (1) the rate of dissolving becomes constant (2) before finally the fluorescence in the bulk levels off (3). The initially more slowly increasing fluorescence is related to the start of core dissolving. At this stage of the process the structure of the polyelectrolyte multilayer may change because of the nascent osmotic pressure coming from dissolved fluorescein molecules. Shortly after the beginning of core dissolution the concentration of fluorescein inside the capsules becomes constant and almost saturated, since a steady state situation between progressing core dissolution and permeation is established. One may further assume a constant concentration gradient between the shell interior and the bulk because the bulk solution can be assumed as being infinitely diluted. Therefore the rate of fluorescein penetration through the polyelectrolyte layers to the bulk becomes constant. Indeed, a linear increase of the fluorescence is observed (2). This state corresponds to the stage of dissolution depicted in Figure 1 E. The slope of the linear region decreases with the number of polyelectrolyte layers. Obviously an increasing number of adsorbed layers reduces the fluorescein penetration. After the core is completely dissolved, the fluorescein concentration inside the shell equilibrates with the bulk. The driving force for diffusion decreases and the release levels off (3).

Figure 4 shows the behaviour of the permeability times thickness as a function of the numbers of layers.

Example 2: Permeability as function of pH

Sodium poly(styrene sulfonate) (Na-PSS, MW ∼ 70 000), poly(allylamine hydrochloride) (PAH, MW ∼ 50 000), dextran (MW ∼ 75 000 and 4 000 000), and bovine serum albumin all labeled with fluorescein isothiocyanate (FITC) were obtained from Aldrich. Hollow polyelectrolyte capsules were fabricated at pH=7 by alternating adsorption of 8 layers PSS/PAH onto MF-particles of a diameter of 5.2 μm ( microparticles GmbH, Berlin), using the filtration protocol (15). The cores were dissolved at pH=1. Washings in 50 mM NaCl followed. Confocal images were taken by means of a confocal laser scanning microscope (TCS Leica). The excitation wavelength was 488 nm. The capsules were suspended into the FITC labeled polymer solution of a concentration of 1 mg/ml.

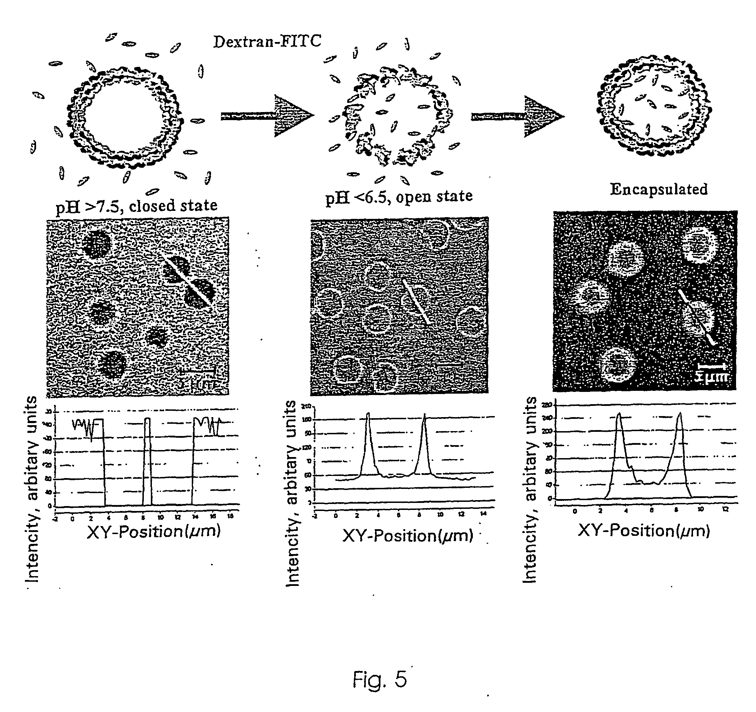

The exclusion properties of hollow polyelectrolyte capsules composed of 8 PSS/PAH layers templated on 5.2 μm melamin formaldehyde (MF) - particles for FITC-labeled dextran (with MW about 75 000) have been studied as a function of pH. Fig.5 (left) provides a confocal image of capsules in the presence of FITC-dextran at pH = 10. The interior of the capsules remains dark, while the background is fluorescent. This proves that at this condition the capsule wall is not permeable for FITC-dextran. Even deformed capsules do not reveal any fluorescence inside. However, at pH = 3 the capsule interior becomes as fluorescent as the bulk (Fig.5, center). This can only be explained by opening of the capsules for FITC-dextran at this low pH value.

It has to be mentioned that the "open" and "closed" states at relatively low pH and high pH, respectively, were observed for more than 90% of the capsules. The open state for FITC-dextran was observed for pH values up to 6. From pH 8 onwards most of the capsules are closed. At a pH value in between, i.e. pH = 7, open and closed capsules were simultaneously present.

The possibility of loading is demonstrated in Fig.5 (right) where the capsules were initially exposed to a FITC-dextran solution at pH = 3. Then the pH was shifted to 10 and the rest of FITC-dextran was removed from the bulk by centrifugation. It is remarkable that the capsules remain filled with fluorescent material as shown by the fluorescence profile through the confocal image. The interior of the capsule observes a bright and constant over time fluorescence while there is no fluorescence signal from solution.

Similar experiments of changing the capsule wall permeability by pH changes and subsequent capsule loading were performed with FITC-dextran of a MW of 2 000 000 and FITC-labeled bovine serum albumin. The results are analogous to those with FITC-dextran of MW 75 000, but the pH value of the transition between the closed and open state differs by 1-1.5 pH units.

Example 3: Controlling capsule wall permeability by the salt concentration

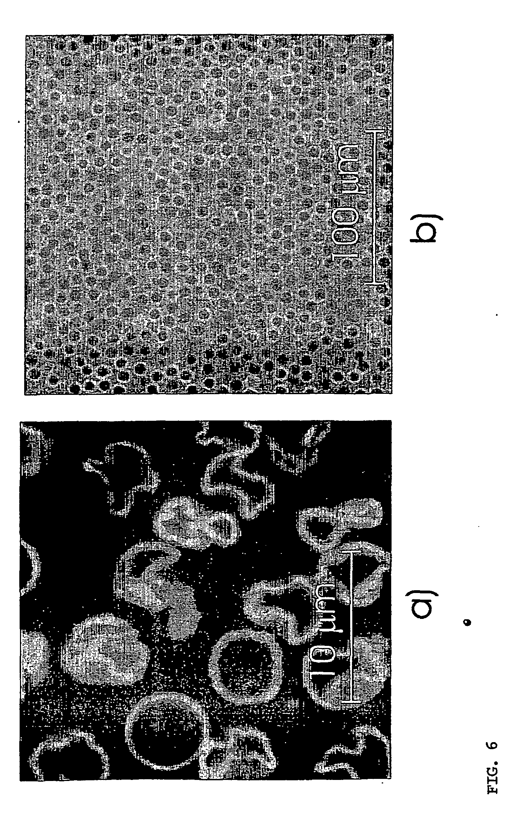

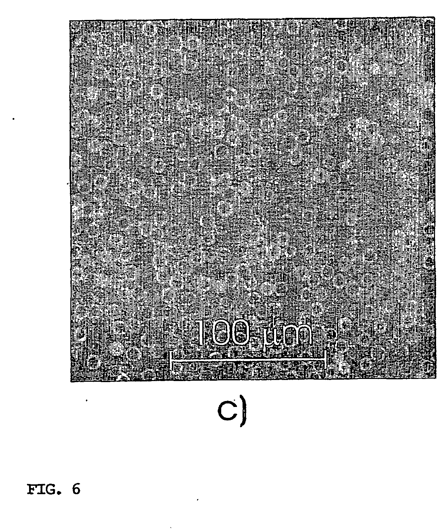

Two different types of polyelectrolyte capsules were used for these permeability experiments. 8 layers of sodium polystyrenesulfonate PSS and polyallylamine PAH were adsorbed on monodisperse templates of diameter 4.7 μm consisting of weakly polymerized melamine-formaldehyde resin. In one batch, core dissolution was conducted at pH 1 and the subsequent washings were performed in the presence of 0.05 M NaCl. This protocol yielded crumpled capsules (Fig. 6a). These capsules were covered by two additional layers and subsequently washed with Millipore water ($\sigma_- < 18$ MΩ cm). After this treatment, the capsules assumed a spherical shape.

The cores in the second batch were dissolved in 0.1 M HCl and washed afterwards with Millipore water thus avoiding the addition of salt. The latter protocol yielded homogeneous population of capsules with a spherical shape (Fig. 6b). The permeability behavior of the two types of capsules was similar. Essentially the results obtained with the second type of capsules will be reported in this example. Only in case of remarkable differences results obtained with capsules having additional two layers will be mentioned.

The permeability of the capsule walls was investigated by means of fluorescence labeled polymers polystyrenesulfonate PSS (120 000 g/mol), polyallylamine PAH (70 000 g/mol), dextrane (55 000 g/mol) and human albumine (70 000 g/mol). Polyallylamine PAH was used which was labeled at every 245th position with rhodamine B. In the Förster resonance energy transfer measurements, polyallylamine PAH labeled at every 120th position with fluoresceine was used. The capsules were added to a $5 \times 10^{-3}$ M solution of the respective probe polymers, which concentration is always expressed in monomer units. The polymer permeation was followed by means of confocal imaging (TCSCN Leica, Germany). The amount of fluorescent molecules inside and outside the capsules was quantified.

The image in Fig.6b shows a confocal scan through the equatorial plane of the capsules, which were incubated with the labeled polymer solution for 24 hours. About 90% of the capsules excluded the polymers. The few with polymer filled capsules were broken and observed large holes through which the polymers could have diffused almost instantaneously into the capsule interior.

A similar experiment was then performed in the presence of 0.1 M NaCl. The confocal image (Fig. 6c) shows, that the interior of all capsules contains approximately the same concentration of the polymer as present in the bulk. This finding is consistent with an increase of the permeability of the polyelectrolyte wall for polymers in the presence of salt. This "wall opening" was observed for positively and negatively charged polymers as well as for the dextrane representing a neutral polymer. Hence, salt induced changes in the structure of the probe polyelectrolytes, such as coiling of the charged species cannot be the responsible mechanism for the penetration. Rather salt-induced changes of the structure and properties of the polyelectrolyte complexes in the wall have to be considered as the cause for the strong increase in permeability. As evident from the images, the fluorescent probe molecules were accumulated on the surface or inside the capsule wall. This was expected for PSS, which is adsorbed onto the positively charged capsule surface, but either for the labeled PAH an exchange of PAH molecules in the capsule wall or an adsorption to the inner PSS layer has to be assumed.

The permeability was next investigated in dependence on the salt concentration. The capsules were mixed with $5 \times 10^{-3}$ M PAH-Rhodamine and increasing concentrations of sodium chloride. The dispersions were incubated for 10 min and the amount of polymer in the interior was compared with the polymer concentration in the bulk. Up to $5 \times 10^{-3}$ M NaCl, no polymer penetration into the capsules was detected, but for higher concentrations, the capsules became increasingly permeable for PAH-Rh. At $2 \times 10^{-2}$ M NaCl, the time of PAH-Rh penetration was estimated by means of confocal imaging overtime. The time between salt addition and complete equilibration of the polymer concentration inside and outside the capsules was app. 6 min for capsules consisting of 8 layers. A slower penetration occurring within 20 min was measured for capsules of 10 layers. Confocal imaging could not follow the kinetics at higher concentrations, because the diffusion and equilibration of the polymer concentration was too fast.

The minimum amount of salt for inducing the polymer penetration was determined by incubating the capsules in polymer solution, at various NaCl concentrations for 24 hours. Then, the percentage of filled capsules was determined by counting in the confocal images the number of filled and empty capsules for app. 100 capsules each (Table 1). While in the $10^{-2}$ M salt solution all capsules contained labeled polymer, in the $0.5 \times 10^{-2}$ M salt solution only 16% of the capsules appeared to be filled within 24 h. In still lower NaCl, the capsules remained impermeable for the polymer. Hence, the permeability increase of the capsules for macromolecules occurred within a rather narrow range of salt concentration between 0.5 and $2 \times 10^{-2}$ M.

It was further examined, whether the rate limiting step of permeation after the addition of salt is provided by salt-induced structure changes in the capsule wall making the wall more permeable or by the diffusion of the polymer through the layer. For this the permeation of PAH $M_n$=70 000 was observed either immediately after the simultaneous addition of $10^{-2}$ M salt (NaCl) and $5 \times 10^{-3}$ M PAH or the sample was incubated for 12 hours in $10^{-2}$ M NaCl and the PAH was added only then. In the second case, it was found that the characteristic permeation time was about 30 min, which was smaller than the characteristic permeation time of about 50 min observed when NaCl and the polymer were added at the same time. These numbers are two fold remarkable. They demonstrate that 1) the permeation of the polymer under the observed conditions is overall slow requiring at least 30 min for concentration equilibration, and 2) that the permeability induction by salt itself requires several minutes.

Regarding the mechanism of the polymer permeation through the capsule wall two extreme situations may be distinguished. Either incubation in the salt solution led to the formation of water-filled pores through the wall across which the polymers can diffuse into and out of the capsule, or the presence of ions in the solution weakens the electrostatic binding between PSS and PAH allowing for diffusion of the polymer through the network via transient bond breaking and re-establishing. Pore formation either induced by pH or by salt was observed for macroscopic flat films composed of weak polyelectrolytes. These relatively large pores had a radius in the order of $10^2$ nm. SFM was thus applied to polyelectrolyte capsules either prepared by drying a capsule dispersion in $H_2O$ or in 0.1 M NaCl applied onto a mica substrate. However, except the presence of some salt crystals no differences in the capsule wall topology were observed. This result argued the formation of large pores as the cause of the permeability increase.

Assuming that the polymer penetration occurs via diffusion trough water-filled pores the apparent pore cross section is of the order of 1000 $nm^2$. This would compare to either one pore of 17 nm radius or to 80 pores of 2 nm each. In any case, it is worth to note that the pore area would constitute a very tiny area fraction of the capsule surface only. From these considerations, it becomes clear that with SFM it is hardly possible to detect these pores even if their existence is sure.

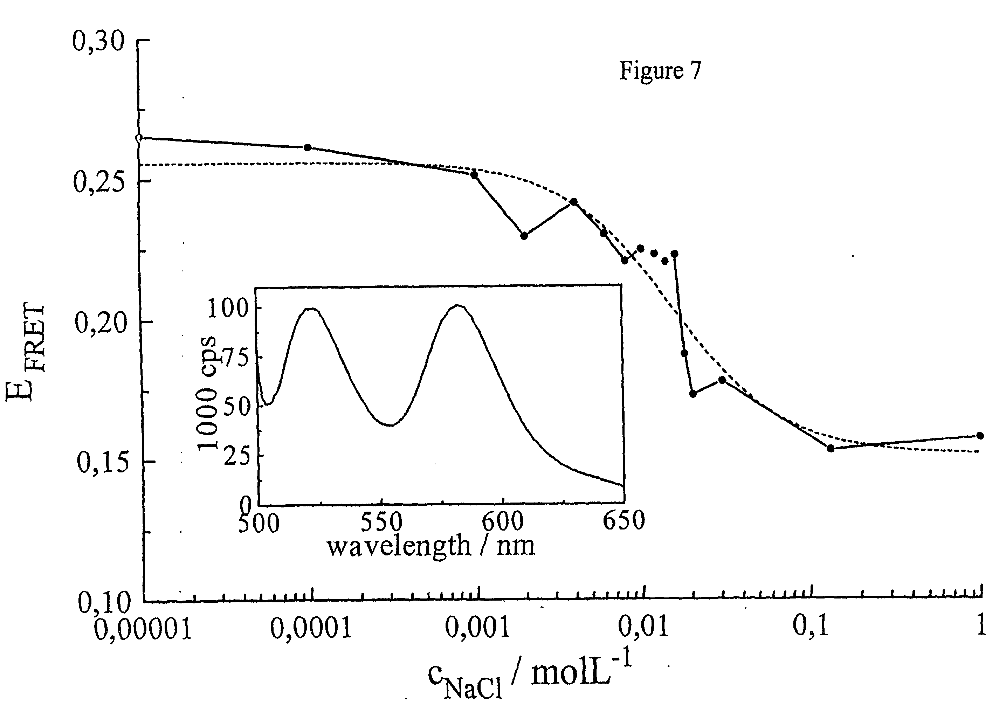

In order to find changes in the capsule wall morphology in dependence on the salt concentration of the bulk electrolyte, Förster resonance energy transfer FRET between fluorescent labeled polyelectrolytes was applied. The capsules were covered by addition of six polyelectrolyte layers in the order PSS, PAH-fluoresceine, PSS, PAH-rhodamine, PSS, PAH. The polymers were adsorbed in presence of 0.5 M NaCl, but afterwards the capsules were extensively washed by water. FRET of the capsule dispersion was measured 5 min after adding NaCl solutions of different concentrations. Excitation was set at 495 nm, where only fluoresceine absorbs light. If rhodamine molecules are located near the fluoresceine, (up to 6 nm) resonance energy transfer takes place and one can observe the rhodamine fluorescence, too (Fig. 7, inset).

The relative transfer efficiency $E_{FRET}$ was defined as $E_{FRET} = (I_{rh} - 0.4I_{fl})/(0.6I_{fl} + I_{rh})$ where $I_{fl}$ is the fluorescence intensity at 522 nm corresponding to the maximum in fluoresceine emission and $I_{rh}$ is the intensity at 582 nm, corresponding to the rhodamine emission.

The factor 0.4 takes into account the fluorescence of the donor at the acceptor emission wavelength. The plot of $E_{FRET}$ against salt concentration shows a clear decrease in the transfer efficiency (Fig. 7), occurring exactly in the concentration range where the capsule wall becomes permeable for PAH. At higher salt concentrations, the $E_{FRET}$ kept almost constant. Hence, it can be concluded that FRET reported remarkable changes in the topology of the polyelectrolyte wall occurring within a narrow salt concentration range around $10^{-2}$ mol/L. The decrease of $E_{FRET}$ with the salt concentration indicates an increase of the distance between the dye molecules of rhodamine and fluoresceine. The FRET signal was completely restored within 5 min when the capsules were expose to water again. These findings are obviously consistent with some reversible swelling of the polyelectrolyte layer.

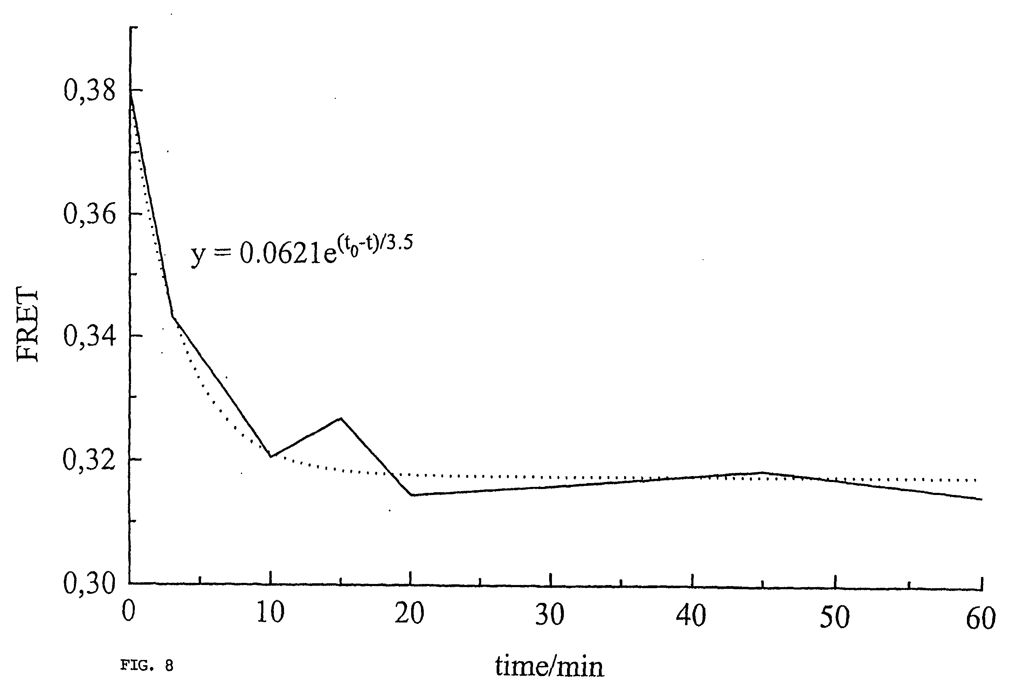

The correlation between the permeability increase of the wall opening and FRET decrease allowed for characterizing the salt-induced capsule wall changes in the absence of a permeating polymer. The latter may have had some influence on the wall properties, especially as much remarkable amounts of the permeating polymer adsorbed to the wall. The capsules were introduced in $1.5 \times 10^{-2}$ M NaCl and the $E_{FRET}$ was measured in dependence on the time (Fig. 8). After 20 min the FRET was saturated at a value of $E_{FRET} = 0.31$. The time scale of the changes was consistent with the permeability change observed by means of confocal microscopy. Both, the time and the degree of the FRET signal change related to wall structure changes increased with the salt concentration.

The mechanism and driving forces causing both layer permeability increase and the decrease of the fluorescence energy transfer occurring in a narrow salt concentration range are discussed below. The rather slow permeation indicates, if water-filled pores are assumed as the basic pathway for permeation, a very small pore area. On the other hand, the charge of the FRET signal can only be understood assuming conformational changes affecting the majority of the labeled polymers. Hence, salt induces layer changes throughout. This is less constant with assuming the much-localized formation of a small number of pores. Therefore we interpret the permeability increase as a result of an increased solubility or interactions of the permeability species with the layer polymers, which are caused by an increased salt concentration.

A higher salt concentration was found to soften the structure of the layer because the ion pairs found between the polyanions and the polycations may partly open, because the free ions available in the bulk would screen the charges in the layer. Layer swelling can thus be understood as a molecular elasticity relaxation of the polymer pairs, leading to a more bulky arrangement of the layer polymers. It is worth to note that at $10^{-2}$ M NaCl the Debye screening length is just 3 nm comparing well to a layer thickness increment of a polyanion/polycation pair. At lower electrolyte concentrations, the electrostatic interactions have a larger range providing an electrostatic interaction over the whole layer.

An important issue for many applications is the reversibility of the permeability switching process. The fact, that the capsules were prepared at high salt concentrations and are nevertheless impermeable for the macromolecules after washing demonstrates the reversibility of the capsule opening and closing. In further experiments the time of closing the capsule wall was determined. A $3 \times 10^{-2}$ M NaCl solution was applied for 30 min to the capsules. Afterwards they were diluted into pure water to app. $5 \times 10^{-3}$ M NaCl. After ten minutes, the capsules became impermeable for the PAH-Rhodamine as was observed by means of confocal imaging. The FRET signal was recovered within 5 min (Fig. 8).

The reversibility of the wall permeability change for polyelectrolytes allows for an easy and soft encapsulation of macromolecules, such as polypeptides, DNA, enzymes, or polymers avoiding any chemical stress caused by aggressive substances, solvents, pH, or heat applied during encapsulation. However, loading of capsules with macromolecules at high salt concentration following by washing with water yielded only small amounts of encapsulated polymer in the interior. Probably, the macromolecules were washed out faster than the wall closed.

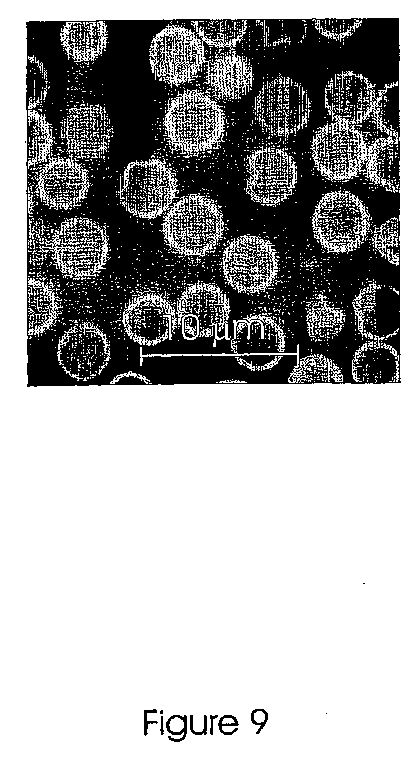

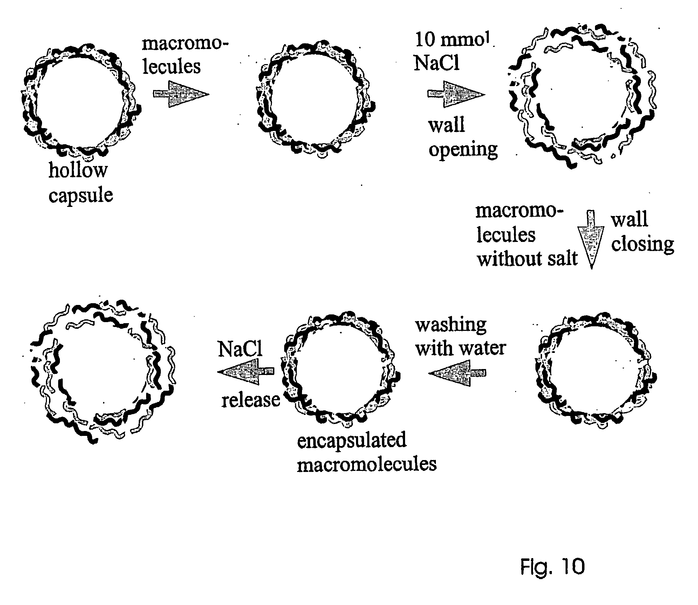

A much higher efficiency of loading was however achieved when the polymer for encapsulation was present in the bulk during closure of the wall induced by salt removal. (Fig. 10). By this way, the capsules were loaded with a $5 \times 10^{-3}$ M PAH-Rh solution (Fig. 9). Taking the fluorescence intensity of the confocal image of the PAH-Rh solution as standard, the amount of encapsulated polymer could be determined. The average of the interior PAH-rho concentration was about 50% of that of the initial solution.

Another important property of the loaded capsules is their release behavior. The loaded capsules were incubated for 30 min either in 2 ml water or in 2 ml of a $3 \times 10^{-2}$ M salt solution. The capsules were removed from the sample by centrifugation and the concentration of the PAH-Rh in both solutions was determined by fluorescence spectroscopy.

The dependence of the rhodamine emission on the salt concentration, was taken into account by means of adjusting the salt free solution afterwards to $3 \times 10^{-2}$ M before the measurement were performed.

While in the pure water the amount of released PAH could not be detected almost all encapsulated PAH-Rh was released in $3 \times 10^{-2}$ M NaCl. Confocal image proved that the interior of those capsules was void of fluorescent polymer again.

Example 4: Temperature dependent permeability

Monodisperse spheres (d = 5.94 $\mu$m) of a weakly polymerized melamine-formaldehyde resin were used as templates (Microparticle GmbH). The particles were covered by alternating adsorption of sodium polystyrene sulfonate PSS (MW 70 000 g mol$^{-1}$, Aldrich) and polyallylamine hydrochloride PAH (MW 70 000 g mol$^{-1}$, Aldrich) from an aqueous solution of $10^{-2}$ M PSS in 0.1 M sodium chloride. After 8 layers the core was dissolved in 0.1 M hydrochloric acid. The decomposition products were removed by washing with acid and an aqueous solution of $10^{-2}$ M NaCl. Hollow polyelectrolyte capsules remained. In order to remove some defects in the capsules wall caused by mechanical stress during the dissolution two additional layers were coated on the capsules after the dissolution of the cores. Afterwards the capsules were washed extensively (8 times) with Milli-Q-water (resistance >18 M$\Omega$ cm$^{-1}$). By this procedure, traces from salt and protons were essentially removed and a pH value of 6 was reached.

For the study of the permeability, fluorescence labeled polymers were used. PAH (MW 70 000, 1 rhodamine molecule per 245 PAH units and MW 15 000, 1 rhodamine per 320 PAH units) and PEi (MW 2 000, 1 fluoresceine molecule per 650 PEI units) were labeled by reaction with rhodamine isothiocyanate or fluoresceine isothiocyanate. Labeled PSS (1 rhodamine molecule per 600 polymer units) was synthesized by copolymerization of styrene sulfonate with methacroyloxyethyl thiocarbamoyl rhodamine B (Polyscience). The MW of the polymer was determined being 120 000 g/mol by gel permeation chromatography. Fluoresceine labeled human serum albumine (MW 69 000) and rhodamine labeled dextrane (MW 40 000 g/mol) were purchased from Aldrich company.

Laser scanning microscopy was performed for the determination of the loading grade of the capsules. An inverse research microscope (Leica, Germany) with either a 40 x or a 100 x oil immersions objective was used. The amounts of polymer/monomer in the interior were determined by integration of the fluorescence intensity inside the capsules. The exact concentration was taken from calibration curves, determined for each fluorescent probe separately. Due to the broad distribution of permeability for the capsules, statistical values were taken from 5 capsules each. Capsules, which could be recognized clearly as broken once (appr. 20%), were excludes from the determination. The temperature dependent measurements were done with an home-made aluminium device on top of the sample, by which the temperature was settled by an electronic controller.

Scanning force microscopy was applied to capsules, dried on air on a mica substrate. The drying process after the heat treatment at 60°C was done by transferring a drop of the capsule solution to the mica surface and drying without any change of the temperature.

4.1 Permeability

The permeability of the capsules was studied for positively (PAH) and negatively charged (PSS), zwitterionic (albumine) and noncharged (dextran) fluorescent polymers (dextran). In general, the capsules were mixed with the probe polymers in a concentration of about 2 x 10⁻³ mol/L with respect to the polymer units and incubated for 15 min. Then, the fluorescence intensity in the center of the capsules was measured by confocal imaging. The fluorescence events within the cross section of the capsule were integrated and compared with the fluorescence events in the bulk solution. Less fluorescence intensity inside than outside the capsules indicates an impermeability of the capsule wall for the probe polymer. Equal fluorescence intensities inside and outside of the capsules at the start of the experiment is caused by fast penetration of the polymers through the capsule wall, mostly observed for broken capsules. In case of dextrane as described below the fluorescence inside the capsules is higher than in the bulk phase indicating an accumulation of the polymer in the interior.

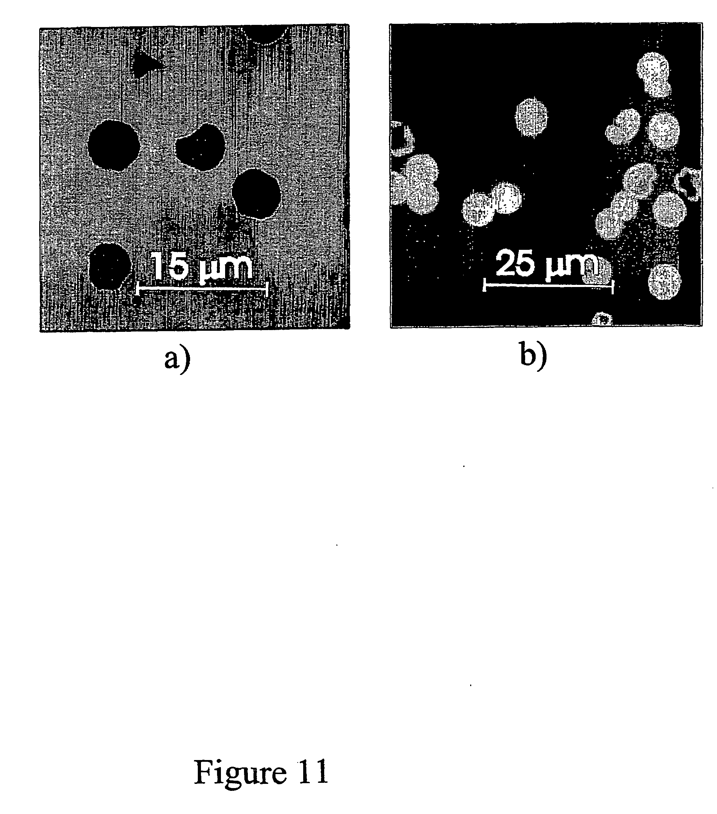

First the permeability of the capsule wall for the probe polymers was studied before any heat treatment. Within 24 hours the majority of capsules were impermeable for charged polyelectrolytes of MW above 60 000 g/mol independent on the sign of the charge. Figure 11 a shows the confocal image for PAH of 70 000 g/mol. In the case of uncharged dextrane an unusual accumulation of the polymer inside of appr. 20% of the capsules was observed (Fig. 11 b), while the other capsules exclude the polymer, too.

Further influence of the length of the polymer chain on penetration was determined. While within 1 hour 80% of the capsules are impermeable for PAH 70 000 g/mol, about 65% were impermeable for PAH 15 000 g/mol and for PEI 2000 all capsules were permeable. Small dye molecules such as fluoresceine, rhodamine and acridine orange can penetrate the capsule wall independent of their charge.

The dependence of the permeability of capsule walls on temperature was followed online by confocal imaging (in situ) by heating capsules in 10° steps from room temperature to 80°C. At each step the temperature was allowed to stabilize for 5 min. The changes in the polymer distribution within the capsules and the bulk solution were similar for each polymer and are demonstrated for heating the capsules in presence of rhodamine labeled PSS. Up to 50°C the polymers was excluded from the capsule wall, but at higher temperature the PSS starts to flow into the capsules until an equilibrium or even a slight excess was reached inside the capsules. The rate of penetration increases with the temperature furthermore. This is a clear proof, that the capsule wall can be opened for large polymer molecules very easy by increase of the temperature.

It was further examined whether the opening is an irreversible process or if the capsule walls can be closed again by reducing the temperature below 50°C. This was investigated by treating the capsules in absence of polymers at different temperature conditions deviating in time of heating and cooling.

Possible mechanisms leading to an increase of capsule wall permeability are discussed below. Besides the fact, that diffusion through a membrane increases generally with temperature due to the higher mobility of the polymer species, the rather strong effect observed in a narrow temperature range indicates structural changes in the polyelectrolyte wall. One possible mechanism could be the opening of pores in the polyelectrolyte membrane as it is described above for treatment with pH and salt for similar systems. It was observed that PSS/PAH capsules shrink at higher temperature by more than 10% in diameter. The mechanical stress at such process could cause also the formation of pores. In order to find such pores, the capsule wall was investigated by high resolution Scanning Force Microscopy after drying capsules on a mica substrate. Three samples were prepared before, during, and after heating to 60°C, but significant differences were not observed apart from the reduction of the capsules in diameter. Despite of this experimental result, the formation of pores can not be fully excluded because of the subsequent drying process can change the capsule structure remarkably.

Another mechanism is the weakening of the electrostatic bounds between polyanions and polycations. This leads to an increased charge density in the film, connected with stronger hydration, swelling and a better diffusion of the polymers through the wall. It was tried to support this mechanism by investigations of the resonance energy transfer in capsules consisting of 14 layers, in which the 10th layer is PAH-fluoresceine and the 12th layer is PAH-rhodamine. The spectra of the solution were taken before, during, and after heating to 60°C for 20 min. The spectra before and after the heating look quite similar, indicating an almost reversible change in the wall structure. The spectrum at 60°C deviates remarkably from the other spectra. The decrease of the rhodamine fluorescence indicates an increase of the averaged distance between fluoresceine and rhodamine molecules or the polymer layers, respectively. In order to exclude simple heating effects as origin of the observed spectra changes, a PAH polymer containing on every 106th position a fluoresceine molecule and on every 345th position a rhodamine molecule (MW 70 000 g/mol) was investigated under identical conditions.

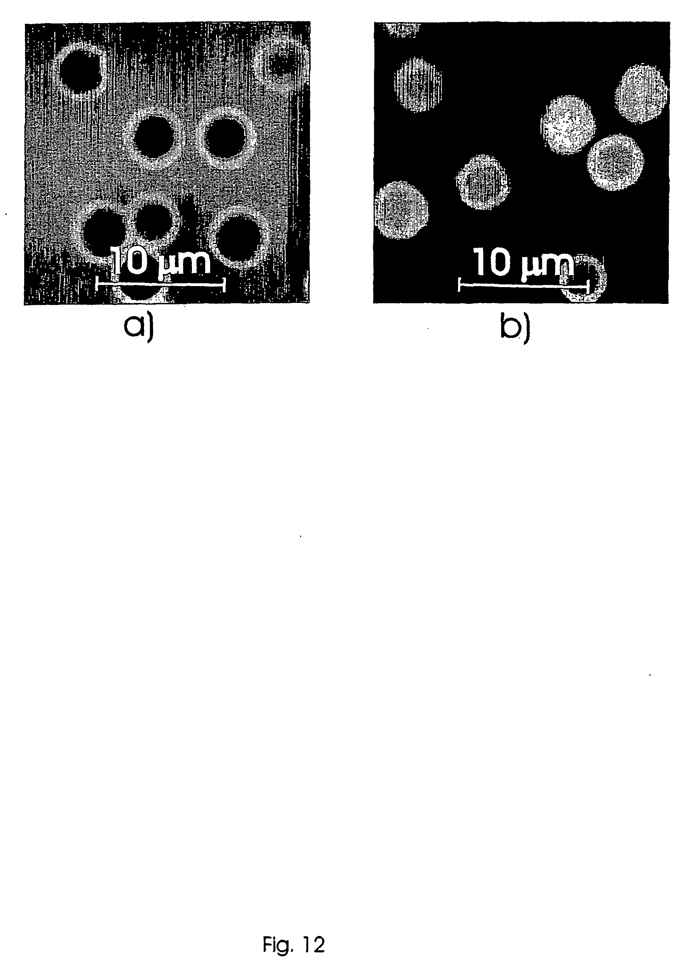

The findings offer a unique possibility to encapsulate macromolecules, since the permeability of capsule walls was found to be controlled by temperature: the capsule wall is opened by heating and surrounding material can stream in. If the capsules are filled with the desired materials the wall can be closed again by cooling down the solution. Afterwards the remaining materials outside, e.g. polymers can be washed away and the enclosed macromolecules remain inside the capsules. The encapsulation was done successfully for all labeled polymers by heating impermeable capsules (Fig. 12a) to 60°C for 20 min. After cooling and washing, the capsules contained the polymer as shown for example in Figure 12b. A quantitative determination of the fluorescence intensity of the original polymer solution and the encapsulated polymer in the interior yielded a 30% of the bulk concentration in the interior. Both probe polymers used, i.e. labeled PSS as well as PAH were captured in the interior indicating a high reversibility of the permeability. Even storage of the filled capsules for more than one month lead only to some loss of polymer. This loss was especially strong with more than 50% for PAH, but zero in case of dextrane.

References

1. G. Decher, Science 1997, 277, 1232
2. B. Miksa, S. Stomkowski, Cooloid Polym. Sci. 1995, 273, 47
3. G. Crotts, T.G. Park, J. Control. Release 1995, 35, 91
4. K.B. Thurmond, T. Kowalewski, K.L. Wooley. J. Am. Chem. Soc. 1997, 1997, 119, 6656.
5. G.B. Sukhorukov, E. Donath, H. Lichtenfeld, E. Knippel, A. Budde and H. Möhwald, Colloids Surfaces A, 1998, 137, 253
6. E. Donath, G.B. Sukhorukov, F. Caruso, S. A. Davis and H. Möhwald, Angew. Chem. Int. Ed. 1998, 37, 2202
7. G.B. Sukhorukov, E. Donath, S.A. Davis, Heinz Lichtenfeld, F. Caruso, V.I. Popov and H. Möhwald, Polym. Adv. Technol., 1998, 9, 759
8. G.B. Sukhorukov, M. Brumen, E. Donath and H. Möhwald, J. Phys. Chem. B 1999, 103, 6434
9. G.B. Sukhorukov, E. Donath, S. Moya, A.S. Susha, A. Voigt, J. Hartman and H. Möhwald, J. Microencapsulation, 2000, 17, 2, 177-185
10. S. Moya, et al. Macromolecules, 2000
11. R. von Klitzig, H. Möhwald, Macromolecules 1996, 21, 6901
12. S. Leporatti et al. Langmuir, 2000
13. a) E. Donath, G. B. Sukhorukov, F. Caruso, S. Davis, H. Möhwald. *Angew. Chem.* **1998**, *110, 2323; Angew. Chemie, Inter. Ed. Eng.,* **1998**, 37, 2201;
14. F. Caruso, R. Caruso, H. Möhwald, *Science,* 1998, 282, 1111.
15. A. Voigt H. Lichtenfeld, G. B. Sukhorukov, H. Zastrow, E. Donath, H. Bäumler, H. Möhwald, *Ind.&Eng.Chem. Res,* **1999**, 38, 4037.
16. G. Decher, J.-D. Hong, *Makromol. Chem., Macromol. Symp.* **1991**, 46, 3211.
17. E. Donath, G. B. Sukhorukov, H. Möhwald, *Nach. Chem. Tech. Lab.,* **1999**, 47, 400.
18. I. L. Radtchenko G. B. Sukhorukov, S. Leporatti, G. B. Khomutov, E. Donath, H. Möhwald, *J. Col. &Int. Sci,* (**2000**), in press.
19. L. Dähne et al., *Angew. Chem.* (**2000**).
20. A.Kabanov, A.Zezin, *Pure Appl. Chem.,* **1984**, 56, 343.
21. B. Philipp, H. Dautzenberg, K.-J-Linow, J. Kötz, W. Dawydoff, *Prog. Polym. Sci.,* **1989**, 14, 91.
22. J. D. Mendelsohn, C.J.Barret, V.V.Chan, A.J.Pal, A.M.Mayes, M.F.Rubner, *Langmuir* (**2000**), in press.

Table 1:

| Percentage of filled capsules after 24 hours incubation time in solutions of PAH-rho, $5 \times 10^{-3}$ M and in presence of different salt concentrations. | | | | | | |
|---|---|---|---|---|---|---|
| Salt concentration /$10^{-4}$ mol/L | 1 | 5 | 10 | 50 | 100 | 200 |
| Percentage of filled capsules/ % | 4.6 | 8.5 | 6 | 16.7 | 100 | 100 |

**Claims**

1. A process for controlling the permeability of polyelectrolyte multilayer capsules comprising the steps:

   (i) providing polyelectrolyte multilayer capsules,
   (ii) modifying the capsule wall permeability of the finished capsules for loading or release of materials into or from the capsules,

   wherein at least one environmental condition selected from pH of the medium, temperature or salt concentration is adjusted or/and varied during storage or/and use of the capsules, wherein, in order to render the polyelectrolyte multilayer capsules impermeable, a pH of the medium of at least 8, a salt concentration of at least $2 \times 10^{-2}$ M and/ or a temperature of at least 50 °C is adjusted or wherein, in order to render the polyelectrolyte multilayer capsules permeable, a pH of the medium of less than 6, a salt concentration of less than $5 \times 10^{-3}$ M and/or a temperature of less than 50 ° C is adjusted.

2. A process according to claim 1, wherein lipids, surfactants, dyes, drug molecules, nanoparticles or/an biopolymers such as polysaccharides, polypeptides, nucleic acids are included as layer constituents.

**Patentansprüche**

1. Verfahren zur Kontrolle der Permeabilität mehrschichtiger Polyelektrolytkapseln, umfassend die Schritte:

   (i) Bereitstellen mehrschichtiger Polyelektrolytkapseln
   (ii) Modifizieren der Kapselwandpermeabilität der fertigen Kapseln zur Beladung oder Freisetzung von Materialien in die oder aus den Kapseln,

   worin wenigstens eine aus dem pH-Wert des Mediums, der Temperatur oder der Salzkonzentration ausgewählte Umgebungsbedingung eingestellt wird oder/und während der Lagerung oder/und der Verwendung der Kapseln verändert wird,
   worin, um die mehrschichtigen Polyelektrolytkapseln impermeabel zu machen, ein pH-Wert des Mediums von wenigstens 8, eine Salzkonzentration von wenigstens $2 \times 10^{-2}$ M und/oder eine Temperatur von wenigstens 50 °C eingestellt wird, oder
   worin, um die mehrschichtigen Polyelektrolytkapseln permeabel zu machen, ein pH-Wert des Mediums von weniger als 6, eine Salzkonzentration von weniger als $5 \times 10^{-3}$ M und/oder eine Temperatur von weniger als 50 °C eingestellt wird.

2. Verfahren gemäß Anspruch 1, worin Lipide, oberflächenaktive Stoffe, Farbstoffe, Wirkstoffmoleküle, Nanopartikel oder/und Biopolymere wie zum Beispiel Polysaccharide, Polypeptide, Nukleinsäuren als Schichtbestandteile eingeschlossen sind.

**Revendications**

1. Procédé pour réguler la perméabilité de capsules multicouches de polyélectrolytes comprenant les étapes:

   (i) de fourniture de capsules multicouches de polyélectrolytes,
   (ii) de modification de la perméabilité de la paroi de capsule des capsules finies pour l'introduction ou la libération de produits dans ou à partir des capsules,

dans lequel on ajuste et/ou on fait varier au moins une condition environnementale choisie parmi le pH du milieu, la température ou la concentration de sels pendant le stockage et/ou l'utilisation des capsules, dans lequel, pour rendre les capsules multicouches de polyélectrolytes imperméables, on ajuste le pH du milieu à au moins 8, la concentration de sels à au moins $2 \times 10^{-2}$ M et/ou la température à au moins 50°C, ou dans lequel, pour rendre les capsules multicouches de polyélectrolytes perméables, on ajuste le pH du milieu à moins de 6, la concentration de sels à moins de $5 \times 10^3$ M et/ou la température à moins de 50°C.

2.  Procédé selon la revendication 1, dans lequel des lipides, des agents tensioactifs, des colorants, des molécules de médicaments, des nanoparticules et/ou des biopolymères comme des polysaccharides, des polypeptides, des acides nucléiques, sont contenus comme constituants des couches.

Fig. 1

Fig. 2

Fig. 3

Fig. 4

Fig. 5

a)

b)

FIG. 6

c)

FIG. 6

Figure 7

EP 1 313 455 B1

$$y = 0.0621e^{(t_0-t)/3.5}$$

FIG. 8

time/min

EP 1 313 455 B1

Figure 9

Fig. 10

Figure 11

a)

b)

Fig. 12

Fig. 13

Fig. 14

The Scheme of encapsulation of macromolecules

The Permeation of High Molecular Weight Molecules

Confocal image of a shell closed for FITC labeled Dextrane (77000) (FITC-Dex) (a) and of a shell with encapsulated FITC-Dex (b) with profiles of fluorescence intensity in a line, crossing the shell

The original shells

Shells with a labeled polymer at low pH or in ethanol

Transfer into water

Shell morphology response on the pH or solvent treatment

AFM image of a shell at ph 4

AFM image of a shell treated with ethanol

AFM image of a shell at pH 8

AFM image of a shell treated with ethanol and washed with water

Fig. 15

EP 1 313 455 B1